# EUROPEAN PATENT SPECIFICATION

(11) **EP 1 535 901 B1**
(45) Date of publication and mention of the grant of the patent: **05.03.2008**
(21) Application number: 02788757.9
(22) Date of filing: 09.12.2002
(51) Int. Cl.: C07C 253/30, C07C 255/50

(54) **PROCESS FOR PRODUCING 4'-BROMOMETHYL-2-CYANOBIPHENYL**
VERFAHREN ZUR HERSTELLUNG VON 4'-BROMMETHYL-2-CYANOBIPHENYL
PROCEDE DE PRODUCTION DE 4'-BROMOMETHYL-2-CYANOBIPHENYLE

(30) Priority: 12.06.2002 JP 2002172016
(43) Date of publication of application: 01.06.2005
(73) Proprietor: Sumitomo Chemical Company, Limited, Tokyo 104-8260 (JP)
(72) Inventor: YAMADA, Seiji, c/o SUMITOMO CHEMICAL CO.,LTD, Kurashiki-shi, Okayama 711-0903 (JP); ISHIHARA, Ken, c/o SUMITOMO CHEMICAL CO., LTD, Kurashiki-shi, Okayama 711-0903 (JP); NAKAMATSU, T., c/o SUMITOMO CHEMICAL CO., LTD, Kurashiki-shi, Okayama 711-0903 (JP)
(74) Representative: Duckworth, Timothy John
(86) International application number: PCT/JP2002/012847
(87) International publication number: WO 2003/106406

(56) References cited:
- EP-A- 0 336 567
- EP-A- 0 853 081
- EP-A- 0 973 729
- EP-A1- 0 709 369
- WO-A1-98/46562
- DE-A1- 19 757 995
- DE-C1- 19 712 339
- US-B1- 6 214 999
- PATENT ABSTRACTS OF JAPAN vol. 2000, no. 12, 3 January 2001 (2001-01-03) & JP 2000 256298 A (SUMIKA FINE CHEMICALS CO LTD), 19 September 2000 (2000-09-19)
- PATENT ABSTRACTS OF JAPAN vol. 2002, no. 07, 3 July 2002 (2002-07-03) & JP 2002 088044 A (SUMITOMO SEIKA CHEM CO LTD), 27 March 2002 (2002-03-27)

## Description

### Technical Field

The present invention relates to a method of producing 4'-bromomethyl-2-cyanobiphenyl, which is useful as a synthetic intermediate for a pharmaceutical product, industrially advantageously.

### Background Art

4'-Bromomethyl-2-cyanobiphenyl is useful as a synthetic intermediate for a pharmaceutical product such as a compound having an angiotensin II antagonistic action as disclosed in JP-A-63-23868. There are various production methods of 4'-bromomethyl-2-cyanobiphenyl, which have been disclosed heretofore.

JP-A-6-192170 describes a method of brominating a 4-methylbiphenyl compound using a brominating agent such as N-bromosuccinimide in a halogenated hydrocarbon solvent in the presence of an azobis compound. However, this method uses expensive N-bromosuccinimide as a brominating agent and is industrially disadvantageous.

JP-A-8-127562 describes a method of producing 4'-bromomethyl-2-cyanobiphenyl by way of a reaction with economical bromine as a brominating agent in the presence of a radical initiator. However, this method uses an equimolar amount of bromine and by-produces hydrogen bromide. Since the by-produced hydrogen bromide inhibits the reaction, a radical initiator needs to be supplemented to complete the reaction. In addition, the method is defective in that further purification is necessary for industrial use because bromine remaining in the reaction system causes coloration of the resulting product.

JP-A-2002-88044 describes a method of removing by-produced hydrogen bromide from the system by conducting the reaction described in the above-mentioned JP-A-8-127562 under reduced pressure. However, this method needs to be performed under reduced pressure and requires a special facility. In addition, since a facility for treating hydrogen bromide, which is a strong acidic gas, is necessary, this is not an industrially advantageous method. Moreover, since half of the bromine atoms contained in bromine does not contribute to the reaction, this method is also economically disadvantageous.

WO99/33788 discloses a method comprising use of bromine produced by a reaction of an aqueous hydrobromic acid solution with hydrogen peroxide as a brominating agent, and reproducing bromine from hydrogen bromide by-produced by bromination by the action of hydrogen peroxide. However, since this method requires use of a 48% aqueous hydrobromic acid solution and a 50% aqueous hydrogen peroxide solution, the water content in the system increases and the volume efficiency becomes degraded. In addition, since highly concentrated aqueous hydrogen peroxide solution, which is dangerous to handle, needs to be used, this method is not an industrially advantageous method.

### Disclosure of the Invention

An object of the present invention is to provide a method capable of producing 4'-bromomethyl-2-cyanobiphenyl industrially advantageously.

The present inventors have conducted intensive studies in an attempt to solve the aforementioned problems and found that the coexistence of an oxidant in the reaction of 4'-methyl-2-cyanobiphenyl with bromine in the presence of a radical initiator enables progress of the reaction without inhibition by hydrogen bromide, because by-produced hydrogen bromide is converted to bromine. Furthermore, they have found that the reaction completes with 0.5 equivalent amount of bromine and 4'-bromomethyl-2-cyanobiphenyl can be produced economically, which resulted in the completion of the present invention. Accordingly, the present invention provides the following.
(1) A method of producing 4'-bromomethyl-2-cyanobiphenyl, which comprises reacting 4'-methyl-2-cyanobiphenyl with bromine in the presence of a radical initiator and an oxidant wherein the oxidant is bromate or chlorate.
(2) The production method of the above-mentioned (1), wherein the oxidant is sodium bromate.
(3) The production method of the above-mentioned (1) or (2), wherein the reaction system contains water.
(4) The production method of the above-mentioned (3), wherein the amount of water contained is a catalytic amount.

The present invention is explained in detail in the following. The synthetic scheme of the production method of the present invention is shown below.

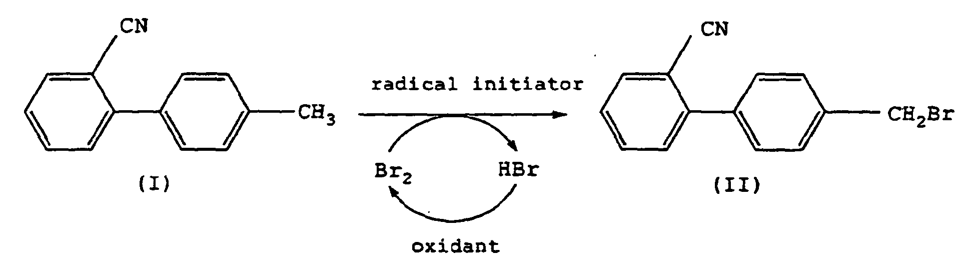

The present invention can be achieved by, for example, reacting 4'-methyl-2-cyanobiphenyl represented by the formula (I) with bromine in a solvent in the presence of a radical initiator and an oxidant. While the order of the addition of the reagents is not particularly limited, it is preferable from the aspect of operability to add bromine or a solution thereof to a mixture of 4'-methyl-2-cyanobiphenyl, a radical initiator and an oxidant, which has been charged in advance in a solvent. Alternatively, bromine and a radical initiator or a solution thereof may be simultaneously added. For smooth progress of the reaction, the reaction mixture is preferably stirred during the reaction.

In the present invention, the amount of bromine used is 0.4-0.7 mol, preferably 0.45-0.60 mol, more preferably 0.52-0.58 mol, per 1 mol of 4'-methyl-2-cyanobiphenyl, which is a starting material. When the amount of bromine used is less than 0.4 mol per 1 mol of 4'-methyl-2-cyanobiphenyl, unreacted starting material tends to remain easily, and when it exceeds 0.7 mol, the by-produced dibromo form tends to increase.

4'-Methyl-2-cyanobiphenyl, which is a starting material in the present invention, can be produced by a known method, such as the methods described in J. Med. Chem. 1991, 34, 2525-2547, JP-A-4-244080, JP-A-4-253949, JP-A-6-9536.

As a radical initiator, azobis compounds, peroxide may be used. Specifically, the azobis compound includes 2,2'-azobis(4-methoxy-2,4-dimethylvaleronitrile), 2,2'-azobisisobutyronitrile, 2,2'-azobis(2-methylbutyronitrile), 2,2'-azobis(2,4-dimethylvaleronitrile); peroxide includes dibenzoyl peroxide, di-t-butyl peroxide and the like, preferably 2,2'-azobis(2-methylbutyronitrile), 2,2'-azobis(2,4-dimethylvaleronitrile), particularly preferably 2,2'-azobis(2-methylbutyronitrile).

The amount of the radical initiator to be used is 0.1-10 mol%, preferably 1-4 mol%, relative to 4'-methyl-2-cyanobiphenyl, which is a starting material. When the amount of a radical initiator to be used is less than 0.1 mol% relative to 4'-methyl-2-cyanobiphenyl, the reaction tends to be slow and when it exceeds 10 mol%, the effect comparable thereto becomes small, which is industrially disadvantageous.

As the oxidant, an oxidant comparatively safe to handle, namely bromates (e.g., sodium bromate, potassium bromate and chlorates (e.g., sodium chlorate, potassium chlorate are used, with preference given to sodium bromate.

The amount of the oxidant to be used only needs to be a theoretical amount necessary for reproducing bromine from by-produced hydrogen bromide or a slightly excess amount thereof, which is 9-20 mol%, preferably 12-17 mol%, relative to 4'-methyl-2-cyanobiphenyl, which is a starting material. When the amount of the oxidant to be used is less than 9 mol% relative to 4'-methyl-2-cyanobiphenyl, bromine is not sufficiently reproduced, and the yield tends to decrease, and when it exceeds 20 mol%, the effect comparable thereto becomes small, which is industrially disadvantageous.

As the solvent to be used in the present invention, halogenated hydrocarbon, alkane having 5 to 7 carbon atoms and aliphatic ester can be mentioned. Specific examples thereof include methylene chloride, ethylene dichloride, carbon tetrachloride, monochlorobenzene, o-dichlorobenzene, bromobenzene, hexane, heptane, cyclohexane, methyl acetate, ethyl acetate, methyl propionate and ethyl propionate. Of these, preferred are monochlorobenzene and ethyl acetate.

The amount of the solvent to be used is generally 0.5 to 20-fold weight, preferably 1 to 20-fold weight, more preferably 1 to 15-fold weight, still more preferably 3 to 15-fold weight, relative to 4'-methyl-2-cyanobiphenyl, which is the starting material. An amount smaller than this range is industrially disadvantageous because the stirring efficiency tends to be poor, and an amount greater than this range is industrially disadvantageous because the reaction tends to be slow.

In the present invention, it is preferable to contain water to the reaction system. Presence of water strikingly increases stirring efficiency and enables smooth progress of the reaction. The water content is 0.01-50% (w/w), preferably 0.05-30% (w/w), of the solvent to be used. Even when the amount of water contained is about the catalytic amount (0.01-2.0% (w/w), preferably 0.05-0.5% (w/w), of the solvent used), the reaction proceeds sufficiently. A water content smaller than 0.01% (w/w) of the solvent to be used is not preferable for industrial production because the stirring efficiency tends to not increase sufficiently, and a water content exceeding 50% (w/w) is not preferable for industrial production because the productivity tends to decrease.

While the reaction temperature varies depending on the radical initiator, it is generally 50-100°C, preferably 50-85°C, more preferably 60-85°C, still more preferably 60-70°C. A reaction temperature lower than this range is industrially insignificant because the reaction tends to be slow and a reaction temperature higher than this range is industrially insignificant because a radical initiator tends to be unstable. The radicals can be also radically produced by photoirradiation of radical initiator. In this case, a mercury lamp may be used. The reaction time can be appropriately determined according to the above-mentioned various reaction conditions (e.g., about 3-10 hr).

4'-Bromomethyl-2-cyanobiphenyl obtained by the above reaction is isolated and purified from a reaction mixture. The method includes, but not limited to, removal of inorganic salt by means of filtration, evaporation of the solvent as necessary and recrystallization from a different suitable solvent according to a conventional method.

4'-Bromomethyl-2-cyanobiphenyl obtained by the present invention can be led to a compound having an angiotensin II antagonistic action by a method described in, for example, JP-A-63-23868, JP-A-6-298684

### Examples

The present invention is specifically explained in the following by referring to Examples, which are not to be construed as limitative.

### Example 1

4'-Methyl-2-cyanobiphenyl (105 g, 0.54 mol) and sodium bromate (12.3 g, 0.082 mol) were added to monochlorobenzene (300 g) and the internal temperature was set to 65°C. After 2,2'-azobis(2-methylbutyronitrile) (2.0 g, 0.010 mol) was added, bromine (45.6 g, 0.29 mol) was added dropwise over 5 hr, and the mixture was warmed for 1 hr. Then, the resulting inorganic salt was filtered off and the reaction solution was cooled to 5°C. The precipitated crystals were collected by filtration to give 4'-bromomethyl-2-cyanobiphenyl (110 g, 0.405 mol)(yield 75%).

### Example 2

Similar reactions and treatments as in Example 1 were performed using 2,2'-azobis(2,4-dimethylvaleronitrile) (2.5 g, 0.010 mol) instead of 2,2'-azobis(2-methylbutyronitrile) to give 4'-bromomethyl-2-cyanobiphenyl (113 g, 0.416 mol) (yield 77%).

### Example 3

Similar reactions and treatments as in Example 1 were performed by adding water (0.3 g) to monochlorobenzene (300 g) to give 4'-bromomethyl-2-cyanobiphenyl (114 g, 0.421 mol) (yield 78%).

### Example 4

4'-Methyl-2-cyanobiphenyl (105 g, 0.540 mol) and sodium bromate (12.1 g, 0.080 mol) dissolved in water (22.9 g) were added to monochlorobenzene (157.5 g) and the internal temperature was set to 80°C. 1/6-fold amount of a solution of 2,2'-azobis(2-methylbutyronitrile) (2.0 g, 0.010 mol) in monochlorobenzene (21 g) (hereinafter solution A) was added. Then, a 5/6-fold amount of solution A and bromine (45.6 g, 0.287 mol) were concurrently added dropwise over 5 hr, and the mixture was warmed at 70°C for 1 hr. The aqueous layer was separated and removed and the reaction mixture was cooled to 5°C. The precipitated crystals were collected by filtration to give 4'-bromomethyl-2-cyanobiphenyl (114 g, 0.416 mol) (yield 78%).

### Example 5

4'-Methyl-2-cyanobiphenyl (105 g, 0.540 mol) and sodium bromate (12.1 g, 0.080 mol) dissolved in water (25.4 g) were added to monochlorobenzene (105.0 g) and the internal temperature was set to 80°C. A 1/6-fold amount of solution A was added. Then, a 5/6-fold amount of solution A and bromine (45.6 g, 0.287 mol) were concurrently added dropwise over 5 hr, and the mixture was warmed at 70°C for 1 hr. The aqueous layer was separated and removed, and the reaction mixture was cooled to 5°C. The precipitated crystals were collected by filtration to give 4'-bromomethyl-2-cyanobiphenyl (114 g, 0.416 mol) (yield 78%).

As shown in the foregoing results, it has been found that, in Examples of the present invention, bromination can be performed using bromine in an amount slightly excess over 0.5 equivalent amount of 4'-methyl-2-cyanobiphenyl, which is a starting material, by the co-presence of sodium bromate as an oxidant.

### Industrial Applicability

According to the production method of the present invention, 4'-bromomethyl-2-cyanobiphenyl useful as a starting material of a pharmaceutical product can be produced industrially advantageously.

According to the production method of the present invention, since the reaction is not inhibited by hydrogen bromide by-produced with the progress of the bromination reaction, the reaction is promoted and can be completed without supplementation of a radical initiator. As a result, only an ultra-trace amount of bromine remains in the reaction system, which in turn affords an advantage in that the resulting product is not colored.

Furthermore, since hydrogen bromide, which is a strong acidic gas, does not have to be removed from the reaction system or be treated thereof, a special facility therefor does not need to be installed. Moreover, since both bromine atoms contained in bromine can be effectively used, which is also economically advantageous. In addition, since the amount of water added as necessary to the reaction system is comparatively small, the volume efficiency can be enhanced, and a dangerous highly concentrated aqueous hydrogen peroxide solution does not need to be used.

## Claims

1. A method of producing 4'-bromomethyl-2-cyanobiphenyl, which comprises reacting 4'-methyl-2-cyanobiphenyl with bromine in the presence of a radical initiator and an oxidant, wherein the oxidant is bromate or chlorate.

2. The production method of claim 1, wherein the oxidant is sodium bromate.

3. The production method of claim 1 or 2, wherein the reaction system contains water.

4. The production method of claim 3, wherein the amount of water contained is a catalytic amount.

## Patentansprüche

1. Verfahren zur Herstellung von 4'-Brommethyl-2-cyanobiphenyl, das das Umsetzen von 4'-Methyl-2-cyanobiphenyl mit Brom in Gegenwart eines Radikalstarters und eines Oxidationsmittels umfasst, wobei es sich bei dem Oxidationsmittel um Bromat oder Chlorat handelt.

2. Herstellungsverfahren gemäß Anspruch 1, wobei es sich bei dem Oxidationsmittel um Natriumbromat handelt.

3. Herstellungsverfahren gemäß Anspruch 1 oder 2, wobei das Reaktionssystem Wasser enthält.

4. Herstellungsverfahren gemäß Anspruch 3, wobei die enthaltene Wassermenge eine katalytische Menge ist.

## Revendications

1. Procédé de production de 4'-bromométhyl-2-cyano-biphényle, comprenant la mise en réaction de 4'-méthyl-2-cyanobiphényle avec du brome en présence d'un amorceur radicalaire et d'un oxydant, dans lequel l'oxydant est un bromate ou un chlorate.

2. Procédé de production selon la revendication 1, dans lequel l'oxydant est le bromate de sodium.

3. Procédé de production selon la revendication 1 ou 2, dans lequel le système réactionnel contient de l'eau.

4. Procédé de production selon la revendication 3, dans lequel la quantité d'eau contenue est une quantité catalytique.
